# EUROPEAN PATENT SPECIFICATION

(11) **EP 4 255 378 B1**
(45) Date of publication and mention of the grant of the patent: **22.07.2026**
(21) Application number: 21816081.0
(22) Date of filing: 29.11.2021
(51) Int. Cl.: A61K 8/34, A61K 8/36, A61K 8/41, A61K 8/68, A61Q 17/00, A61Q 19/10, A61Q 11/00, A61Q 5/00, A61K 8/42, A61K 8/60

(54) **TOPICAL SANITISING COMPOSITION COMPRISING MINIMAL AMOUNTS OF AN ANITMICROBIAL LIPID**
TOPISCHE DESINFIZIERENDE ZUSAMMENSETZUNG
COMPOSITION DE DÉSINFECTION TOPIQUE

(30) Priority: 02.12.2020 IN 202021052554; 19.01.2021 EP 21152232
(43) Date of publication of application: 11.10.2023
(73) Proprietor: Unilever IP Holdings B.V., 6708 WH Wageningen (NL); Unilever Global IP Limited, Wirral, Merseyside CH62 4ZD (GB)
(72) Inventor: GHOSH, Rimpa, Whitefield, Bangalore 560 066 (IN); MAJUMDAR, Amitabha, Whitefield, Bangalore 560 066 (IN); MALLEMALA, Prathyusha, Whitefield, Bangalore 560 066 (IN); WASKAR, Morris, Whitefield, Bangalore 560 066 (IN)
(74) Representative: Unilever Patent Group
(86) International application number: PCT/EP2021/083408
(87) International publication number: WO 2022/117514

(56) References cited:
- WO-A1-2018/166758
- WO-A1-2020/052916
- WO-A1-2020/126347
- US-A1- 2013 280 312
- US-A1- 2016 324 754
- US-A1- 2019 290 662
- DATABASE GNPD [online] MINTEL; 10 September 2020 (2020-09-10), ANONYMOUS: "Acne-Repairing Mask", XP055820081, retrieved from https://www.gnpd.com/sinatra/recordpage/8094441/ Database accession no. 8094441

## Description

### Field of the invention

The present invention relates to a sanitising composition for topical use.

### Background of the invention

People try to take good care of their skin as well as that of their pets. Specific skin related issues that people care about include, good skin health free of infections, good skin tone and adequate moisturization.

Likewise, people want their oral cavity including the gums and teeth to be free of problems like cavities, tartar, gingivitis, caries, bad breath also known as halitosis and plaque.

People are also concerned with hair and scalp care. They generally prefer to have thick and long hair with minimum hair fall. Dandruff is a commonly occurring scalp problem for which a fungal microorganism has been implicated.

A good health of skin, oral cavity and scalp are typically achieved by keeping them free of infections. One way to tackle infections is to treat it using antimicrobials after the infection has set in. Another approach is to leave a minimal amount of antimicrobial composition on the surface like e.g. skin of the hands, so that any invading microorganisms like e.g. bacteria are killed or inactivated so as to minimize spread of disease. The present inventors with their extensive knowledge in the area of skin friendly actives and of antimicrobial activity have come up with a unique combination of a polyhydroxy compound, an antimicrobial lipid and tetrahydroxypropyl ethylene diamine to provide synergistic antimicrobial activity.

There are various topical antimicrobial composition disclosed in the art.

US 3,050,467 (Horowitz et al. 1962) discloses an antimicrobial cleansing composition consisting essentially of a mixture of a water-soluble soap and a silver salt of partially depolymerized alginic acid. The composition provides synergistic antimicrobial activity.

US 2008/014247 (Lu et al., 2008) discloses a composition having metal containing material, stearic acid and a pharmaceutically acceptable carrier to treat conditions caused by gram-positive, gram-negative, fungal pathogens and/or antibiotic-resistant bacteria. It further provides a method for inhibiting biofilm proliferation. The metal containing material can be silver.

The use of tetrahydroxypropyl ethylenediamine is also known in the art.

US 2009/0227683 (Liebel et al., 2009), discloses compositions for providing an analgesic effect to skin. The examples describe a compositon containing tetrahydroxypropyl ethylenediamine, glycerin, D-Panthenol, Nipagin M and Nipasol M.

US 2016/0324754 (Cure et al., 2016) discloses a shaving cream composition comprising: water, carbomer, ethylhexylpalmitate, glycerin, glyceryl acrylate/acrylic acid, isododecane, stearic acid, butylene glycol, ethyl alcohol, cetearyl alcohol, ceteareth-20,propanediol, Brassica Campestris, phenoxyethanol, ethylhexyl glycerin, tetrahydroxypropyl ethylenediamine, Aloe Barbadensis, carboxymethylhydroxyehylcellulose, bisabolol, PEG-45M, and tocopheryl acetate.

Glycerol and panthenol are polyhydroxy compounds that are used as moisturisers in topical compositions.

WO20126347 (Unilever) relates to an antimicrobial composition for malodor and oral biofilm inhibitions benefits and provides an antimicrobial composition comprising a. 0.1 to 10% by weight of tetrahydroxypropyl ethylenediamine (THPE) and, b. 0 001 to 10% by weight of at least one compound selected from a biphenol.

WO18166758 (Unilever) relates to an antimicrobial composition, especially one which provides synergistic anti-dandruff or anti-acne efficacy. This is achieved through a judicious combination of select essential oil actives chosen from thymol, or terpineol and certain antimicrobial lipids selected from sapienic acid, palmitoleic acid, sphingosine, dihydrosphingosine, phytosphingosine, and 6-hydroxysphingosine. These compositions can be delivered through very many different types of personal care products e.g. shampoo, conditioner; face wash or hand wash product; or a leave on cream/lotion.

Despite efforts thus far, people are always on a look out for new technologies e.g. actives or combination of actives that delivers improved antimicrobial benefit. Further, it is preferred if minimal amounts of known antimicrobial actives are used.

Therefore, antimicrobial compositions and actives that deliver an antimicrobial benefit, remains a topic of interest.

### Summary of the invention

The inventors have developed a topical composition that provides very effective antimicrobial efficacy by combining (i) polyhydroxy compound selected from a saccharide, a polyol or combinations thereof, (ii) an antimicrobial lipid and (iii) tetrahydroxypropyl ethylenediamine, wherein the antimicrobial lipid and THPE are present in a weight ratio from 1:10,000 to 1:10.

The topical composition of the present invention can suitably be used in a method of disinfecting a surface, said method comprising application of the topical composition onto the surface.

The invention also relates to the use of the topical composition for sanitising skin, hair or the oral cavity.

The invention also relates to use of a polyhydroxy compound selected from a saccharide, a polyol or combinations thereof, as an antimicrobial activity promoter in a composition comprising a combination of antimicrobial lipid and tetrahydroxypropyl ethylenediamine (THPE).

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated and may be abbreviated as "wt%". Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "x to y" are understood to include x and y. When for a specific feature multiple preferred ranges are described in the format "x to y", it is understood that all ranges combining the different endpoints are also contemplated.

For the purpose of the present invention, the terms 'sanitising' and 'antimicrobial' may be used interchangeably and the denote the same meaning.

Accordingly, a first aspect of the present invention relates to a topical composition comprising:
a) polyhydroxy compound selected from a saccharide, a polyol or combinations thereof;
b) antimicrobial lipid; and
c) tetrahydroxypropyl ethylenediamine (THPE), wherein the antimicrobial lipid and THPE are present in a weight ratio from 1:10,000 to 1:10.

Polyhydroxy compound as per this invention refers to compounds that contain two or more hydroxy groups. The polyhydroxy compounds that may be used in the present invention may be selected from the groups of saccharides or polyols.

Preferred saccharides may be monosaccharides, disaccharides or polysaccharides. Preferred monosaccharides for use in the present invention are one or more of glucose, fructose, galactose, mannose or ribose; more preferably glucose, fructose or galactose. Preferred disaccharides for use in the present invention are selected from one or more of sucrose, lactose, lactulose, maltose, turanose or tehalose; more preferably sucrose, lactose, lactulose or trehalose. By a polysaccharide as per the present invention is meant a compound having more than two saccharide units and thereby also includes oligosaccharides. Preferred polysaccharides for use in the present invention are selected from one or more of fructose oligosaccharide, glucose oligosaccharide or saccharide isomerate; more preferably it is saccharide isomerate. Saccharide isomerate is commercially available under trade name 'Pentavitin^{®}' (from DSM), under trade name Waterin (from Clariant), under trade names 'EPS3 Powder', 'EPS4 Powder', 'EPS5 Powder' and 'EPS15 Powder' (all from Codif) and under trade name 'Hyanify' (from Lipotec S.A.).

The most preferred saccharide for use in the composition of the present invention is selected from one or more of fructose, sucrose, trehalose or saccharide isomerate.

Polyols as per this invention are compounds that contain more than one alcohol group. The preferred polyols as per this invention are glycerol or panthenol.

Panthenol (2,4-Dihydroxy-N-(3-hydroxypropyl)-3,3-dimethylbutanamide) is the alcohol analogue of pantothenic acid (vitamin B5).

The term "antimicrobial lipid" as used herein refers to fatty acids, monoglycerides, sphingolipids and ceramides comprising a linear C₁₀₋₂₄ saturated or unsaturated aliphatic chain, which aliphatic chain may be substituted with one or more hydroxyl groups.

The term "sphinoglipids" as used herein refers to aliphatic amines, containing two or three hydroxyl groups, and often a distinctive trans-double bond in position 4.

The term "ceramides" as used herein refers to a fatty acid ester of a sphingolipid in which the amine group of a sphingoid base is N-acylated with a fatty acid moiety.

Examples of sphingolipids that may be employed as antimicrobial lipid in accordance with the present invention include sphingosine, dihydrosphingosine, phytosphingosine, 6-hydroxysphingosine. Most preferably, the sphingosine used is phytosphingosine.

Examples of antimicrobial fatty acids that may be employed in accordance with the present invention include lauric acid, myristic acid, palmitic acid, stearic acid, arachidic acid, myristoleic acid (9-cis-tetradecenoic acid), palmitoleic acid (9-cis-hexadecenoic acid), sapienic acid (cis-6-hexadecenoic acid), oleic acid and cis-vaccenic acid (cis-11-octadecanoic acid). Preferred antimicrobial fatty acids are monounsaturated fatty acids. Most preferably, the antimicrobial fatty acid is selected from palmitoleic acid, sapienic acid and combinations thereof.

Examples of antimicrobial monoglycerides that may be employed in accordance with the present invention include monoglycerides comprising a residue of a fatty acid selected from lauric acid, myristic acid, palmitic acid, stearic acid, arachidic acid, myristoleic acid, palmitoleic acid, sapienic acid, oleic acid and cis-vaccenic acid. Preferred antimicrobial monoglycerides are glycerol monopalmitoleate, glycerol monosapienate and combinations thereof.

Preferably, the antimicrobial lipid employed in accordance with the present invention is selected from fatty acids, sphingolipids and combinations thereof.

According to a particularly preferred embodiment, the antimicrobial lipid that is employed in accordance with the present invention is preferably selected from sapienic acid, sphingosine, dihydrosphingosine, phytosphingosine, 6-hydroxyosphingosine, palmitoleic acid and combinations thereof. Most preferably, the antimicrobial lipid is phytosphingosine.

The antimicrobial lipid is preferably contained in the topical composition in a concentration 0.0001 to 5 wt%, more preferably 0.0005 to 1 wt% and most preferably 0.001 to 0.1 wt%.

The polyhydroxy compound is preferably present in the topical composition in a concentration 0.01 to 25 wt%, more preferably 0.1 to 10 wt% and most preferably 0.3 to 3 wt%.

THPE is preferably present in the topical composition in a concentration 0.01 to 10 wt%, more preferably 0.05 to 5 wt% and most preferably 0.1 to 2 wt%.

The polyhydroxy compound and THPE are preferably present in the topical composition in a weight ratio in the range from 1:20 to 20:1, more preferably in the range from 1:10 to 10:1 and most preferably in the range from 1:5 to 5:1.

Preferably, the antimicrobial lipid and THPE are present in a weight ratio in the range from 1:10,000 to 1:10, more preferably in the range from 1:5000 to 1:20 and even more preferably in the range from 1:5000 to 1:50, further more preferably in the range from 1:2500 to 1:75 and most preferably in the range of 1:1000 to 1:100.

Preferred combinations of a) polyhydroxy compound selected from a saccharide, a polyol or combinations thereof; b) antimicrobial lipid; and c) tetrahydroxypropyl ethylenediamine (THPE) are as follows:
- Glycerol + Phytosphingosine + THPE,
- Panthenol + Phytosphingosine + THPE,
- Glycerol + Panthenol + Phytosphingosine + THPE,
- Glycerol+ Lauric acid + THPE,
- Fructose + Lauric acid + THPE,
- Fructose + Phytosphingosine + THPE,
- Sucrose + Phytosphingosine + THPE,
- Sucrose + Lauric acid + THPE,
- Fructose oligosaccharide (FOS) + Lauric acid + THPE,
- FOS + Phytosphingosine +THPE.

Besides the polyhydroxy compound, the antimicrobial lipid and the THPE, the topical composition may contain other cosmetically acceptable ingredients such as solvents, thickeners, gelling agents, emollients, UV absorbers, moisturisers, penetration enhancers, essential oils, preservatives, anti-oxidants, surfactants, vitamins, pigments and fragrances.

According to a preferred embodiment, the topical composition is water based. Preferably, the topical composition comprises 0.1 to 99.9 wt% water, more preferably 1 to 99 wt% water and most preferably 5 to 95 wt% water.

The topical composition of the present invention preferably is a lotion, a cream, a deodorant, a hand sanitizer or a body spray.

The composition could be delivered on to the topical surface of the body as a leave-on product or a wash-off product. By a leave-on product is meant a product that is applied to the topical surface of the body and left thereon to deliver its desired function till it may be substantially removed by washing and/ or rinsing usually during the course of the next personal wash activity like a shower or a bath. By a wash off product is meant a product used for personal washing which contains sufficient amount of surfactants meant to aid in cleaning of the body with copious amounts of water. After application of such a product on to the skin usually accompanied by large amounts of lather, the topical surface may be rinsed with sufficient amount of water to leave it substantially free of the product.

The composition as per this invention is preferably a leave- on product.

The composition as per the present invention may be used in a wash off product for cleaning the oral cavity, skin or hair e.g. those formulated as a toothpaste, toothpowder, a mothwash, a shampoo, a hair conditioner, a soap bar, a body or face wash formulated in the form of a gel, an emulsion or a liquid. Preferably the composition is formulated as a leave-on product for care of the skin or as a deodourant product for application on the underarm. Preferably the composition of the invention comprises a cosmetically acceptable base. The cosmetically acceptable base to deliver a skin care product or a deodorant product may have the following optional ingredients.

### Skin Care

The composition of the invention may be used for skin care. By skin care is meant that the composition is applied on the skin and left thereon till the person goes for a shower or a bath usually after several hours or after about a day. The cosmetically acceptable base is generally present in an amount ranging from 10 to 99.9 wt%, more preferably from 40 to 85 wt% of the composition. It is particularly preferred that the cosmetically acceptable base includes water. Water is preferably included in an amount from 30 to 90 wt%, more preferably from 30 to 85 wt%, most preferably from 30 to 80 wt%. Besides water, suitable carrier classes include silicones, polyhydric alcohols, hydrocarbons, triglycerides and thickening powders.

The skin care composition of the invention may be in any form including toners, lotions, creams, mousses, scrub, serum or gel that is suitable for topical application to the skin. It is preferred that the composition is a skin lotion or a cream.

The composition may comprise an emollient oil that act as a co-solvent. Suitable emollient oils include, for example, ester of alkoxylated aromatic alcohol with fatty carboxylic acid, esters of polyglycols or diols with fatty carboxylic acid such as caprylic/capric triglyceride, ester of fatty alcohol and fatty acid, alkoxylated derivative of benzyl alcohol and mixtures thereof. Preferably the emollient oil is caprylic/capric triglyceride.

Typically, such compositions comprise co-solvent in an amount from 0.01 to 10%, more preferably from 0.1 to 8%, most preferably from 1 to 6%, based on the total weight of the sunscreen composition and including all ranges subsumed therein.

The composition may additionally comprise sunscreen agents such as inorganic sunscreens or an organic sunscreen. Examples of organic sunscreens are zinc oxide, titanium dioxide, iron oxide, silica such as fumed silica. The composition of the invention may comprise a UV-A sunscreen agent selected from the group consisting of a dibenzoylmethane derivative, a triazine derivative, a benzophenone derivative and mixtures thereof. The composition of the invention may also comprise a UV-B sunscreen agent. Suitable UV-B sunscreen agent of the invention is selected from the group consisting of a benzophenone, an anthranilate, a salicylate, a cinnamate, a camphor, benzylidene malonate, a triazone, and derivatives thereof.

A skin lightening agent may also be incorporated into the composition of the invention.

Skin care products also include sanitizers that ensure almost instant kill of microorganisms present on the skin. Sanitizers preferably comprise 20 to 90 wt%, preferably 30 to 80 wt% C₂ to C₄ monohydric alcohols wherein said monohydric alcohols are selected from ethanol, isopropyl alcohol or combinations. The sanitizer composition may comprise a combination of ethanol and isopropyl alcohol. Further more preferably, the composition comprises 40 to 75 wt%, still more preferably 55 to 70 wt% and optimally 60 to 70 wt % of the C₂ to C₄ monohydric alcohols.

Preferably, sanitizer compositions may contain 30 to 80 wt%, more preferably 40 to 70 wt%, further more preferably 50 to 68 wt% and most preferably 60 to 65 wt% ethanol. Sanitizer may include certain additional polyols. Additional polyols in the composition according to the invention are preferably sorbitol, glycerol, polyethylene glycol, propylene glycol and combinations thereof.

Sanitizers preferably comprise 0.01 to 1 wt% of a thickening agent. More preferably the composition comprises 0.05 to 0.8 wt% of a thickening agent, most preferably 0.08 to 0.2 wt% of a thickening agent. Water is generally present in a sanitizer composition from 10 to 40 wt%. More preferably the composition comprises 12 to 35 wt% of water, most preferably 25 to 30 wt% of water.

### Deodorant products

A specific class of skin care compositions is what is known as deodourant compositions. These compositions are usually applied to areas of the skin which generate body odour like the underarm region. These products work by either masking the odour or by ensuring that odour causing molecules are not generated or by killing or inactivating the odour causing microorgansims. A class of such products are also known as antiperspirant products as they reduce or minimize the amount of sweat that is generated by the person.

These products can be applied cosmetically and topically to the skin, broadly speaking, by one of two methods. In one method, sometimes called a contact method, a composition is wiped across the surface of the skin, depositing a fraction of the composition as it passes. In the second method, sometimes called the non-contact method, the composition is sprayed from a dispenser held proximate to the skin. The spray can be developed by mechanical means of generating pressure on the contents of the dispenser, such as a pump or a squeezable sidewall or by internally generated pressure arising from a fraction of a liquefied propellant volatilising, the dispenser commonly being called an aerosol.

There are broadly speaking two classes of contact compositions, one of which is liquid and usually applied using a roll-on dispenser or possibly absorbed into or onto a wipe, and in the second of which the desired active is distributed within a carrier liquid that forms a continuous phase that has been gelled. In one variation, the carrier fluid comprises a solvent for the desired active and in a second variation, the active remains as a particulate solid that is suspended in an oil, usually a blend of oils.

### Stick or soft solid compositions:

Many different materials have been proposed as gellant for a continuous oil phase, including waxes, small molecule gelling agents and polymers, They each have their advantages and of them, one of the most popular class of gellant has comprised waxes, partly at least due to their ready availability and ease of processing, including in particular linear fatty alcohol wax gellants. A gelled deodourant composition is applied topically to skin by wiping it across and in contact with the skin, thereby depositing on the skin a thin film.

### Roll-on:

Liquid compositions that are applicable from a roll-on broadly speaking can be divided into two classes, namely those in which an active is suspended in a hydrophobic carrier, such as a volatile silicone and those in which the active is dissolved in a carrier liquid. The latter has proven to be more popular. There are mainly two sorts of dissolving carrier liquid, namely carriers that are predominantly alcoholic, which is to say the greater part of the dissolving carrier fluid comprises ethanol and the second class in which the carrier liquid is mainly water. The former was very popular because ethanol is a mild bactericide in its own right, but its popularity waned because it stings, especially if the surface onto which the composition has been applied has been damaged or cut, such as can easily arise during shaving or other de-hairing operations.

The second class of formulations that is an alternative to alcoholic formulations comprise a dispersion of water-insoluble or very poorly water soluble ingredients in an aqueous solution of the active. Herein, such compositions will be called emulsions. Roll-on emulsions commonly comprise one or more emulsifiers to maintain a distribution of the water-soluble ingredients.

### Aerosol compositions:

Deodourant compositions may be delivered through an aerosol which comprises a propellant in addition to the other ingredients described hereinabove.

Propellants herein generally accord with one of three classes; i) low boiling point gases liquifided by compression, ii) volatile ethers and iii) compressed non-oxidising gases.

Class i) is conveniently a low boiling point material, typically boiling below -5°C, and often below -15°C, and in particular, alkanes and/or halogenated hydrocarbons. This class of propellant is usually liquefied at the pressure in the aerosol canister and evaporates to generate the pressure to expel the composition out of the canister. Examples of suitable alkanes include particularly propane, butane or isobutene. The second class of propellant comprises a very volatile ether of which the most widely employed ether hitherto is dimethyl ether. This propellant can advantageously be employed at relatively low weight ratio of propellant to base formulation, for example to as low as 5:95. It can also be employed in admixture with, for example, compressible/liquefiable alkane gasses. The third class of propellant comprises compressed non-oxidising gasses, and in particular carbon dioxide or nitrogen. Inert gases like neon are a theoretical alternative.

Skin care compositions may also comprise other ingredients which are common in the art to enhance physical properties and performance. Suitable ingredients include but are not limited to humectants, thickeners, opacifiers, binders, colorants and pigments, pH adjusting agents, preservatives, optics, perfumes, viscosity modifiers, biological additives, buffering agents, conditioners, essential oils and skin benefit agents including anti-inflammatory agents, cooling agents, antiperspirant agents, anti-aging agents, anti-acne agents, anti-microbial agents and antioxidants.

Another aspect of the present invention relates to a method of disinfecting a surface comprising the step of applying onto the surface a topical composition as described herein before. The topical composition may be applied, for instance, by spraying, brushing or rubbing.

Typically, the topical composition is applied in an amount of 0.01-100 mg/cm², more preferably in an amount of 0.1-10 mg/cm² and most preferably an amount of 1-5. mg/cm².

According to one advantageous embodiment of the present method, the surface onto which the topical composition is applied is skin or hair.

According to another advantageous embodiment, the surface onto which the topical composition is applied is selected from the inner lining of the oral cavity, tongue and teeth.

Yet another aspect of the invention relates to the use of the topical composition as described herein before for sanitising skin, hair or the oral cavity.

Preferably, the aforementioned use comprises topical application of the composition.

The method and use claimed in the present invention may be for therapeutic or non-therapeutic applications, preferably for non-therapeutic application.

In a further aspect, the invention relates to use of a polyhydroxy compound selected from a saccharide, a polyol or combinations thereof, as an antimicrobial activity promoter in a composition comprising a combination of antimicrobial lipid and tetrahydroxypropyl ethylenediamine (THPE). These polyhydroxy compounds surprisingly promote the antimicrobial activity of the other components in the composition, even though they are typically not antimicrobially active themselves.

All features preferred with regard to the composition of the first aspect of the invention are likewise preferred in this use of a polyhydroxy compound according to the invention. The polyhydroxy compound is preferably selected from glycerol, panthenol, sucrose and combinations thereof.

The invention will now be illustrated with the help of the following non-limiting examples.

### Examples

The following materials were used in the examples described below: glycerol (Merck cat. No. G9012), panthenol (Sigma cat. No. 76200), sucrose (Sigma cat. No. S8501), fructose (Sigma cat. No. F0127), FOS (Tata NQ cat. No. FOSSENCE^{™} Powder P95), THPE (Sigma-Aldrich cat. No. 122262), phytosphingosine (Evonik), lauric acid (Lobachemie cat. No. 0435100500) sapienic acid (Chemscene cat. No. CS-0105451), sphingosine (Sigma cat. No S9666), dihydrosphingosine (Sigma cat. No D6783) and palmitoleic acid (Sigma cat. No P9417).

The antimicrobial activity of compositions containing one or more components selected from polyhydroxy compound (glycerol or panthenol), THPE and antimicrobial lipid (phytosphingosine) was determined using the following assay:
- Prepare a M9 Minimal Medium by adding 200 mL sterile Difco^{™} M9 Minimal Salts, 5x (comprising disodium phosphate, monopotassium phosphate, sodium chloride and ammonium chloride) to 750 mL sterile purified water, adjusting the final volume to 1 L, and aseptically adding 20 mL filter-sterilized 20% glucose solution and 2 mL sterile 1.0 M MgSO₄ solution.
- Inoculate *Staphylococcus epidermidis ATCC 12228 (Chromachemie)* in 10 ml of M9 Minimal Medium + 10% Tryptic Soy Broth (TSB) medium and
   (a) No addition
   (b) 1 wt% glycerol
   (c) 1 wt% panthenol
   (d) 1 wt% glycerol + 1 wt% panthenol
- Incubate at 37°C under shaking conditions for 72 hrs
- After 72 hrs, collect the supernatants from above ferments by centrifugation and pass it through 0.22 µm filter (Filtration is to remove residual bacteria from the culture supernatant). During incubation *S*. *epidermidis* can utilize glycerol and/or panthenol to produce metabolites which can inhibit growth of pathogens.
- Inoculate *Staphylococcus aureus* ATCC 6538 (Chromachemie) in media along with culture supernatant (collected as shown above)
   (1) No addition
   (2) 1 wt% THPE
   (3) 2 wt% THPE
   (4) 0.0004 wt% phytosphingosine
   (5) 1 wt% THPE + 0.0004 wt% phytosphingosine
- Incubate at 37°C without shaking for 16 hrs
- Measure the absorbance at 600 nm after 16 hrs (Absorbance values represent bacterial growth - Higher absorbance value means higher bacterial growth).

The X in the cells of the tables below indicates that the particular ingredient is included in the amount indicated above.

The results are shown in Tables below:

**Table 1**

| **Ex.** | **Glycerol** (1 wt%) | **THPE** (1 wt%) | **Phytosphingosine** (0.0004 wt%) | **Absorbance** | **SD** |
|---|---|---|---|---|---|
| A | X | - | - | 0.67 | 0.07 |
| B | - | X | - | 0.87 | 0.13 |
| C | X | X | - | 0.91 | 0.07 |
| D | X | - | X | 0.45 | 0.12 |
| 1 | X | X | X | 0.12 | 0.01 |

**Table 2**

| **Ex.** | **Panthenol** (1 wt%) | **THPE** (1 wt%) | **Phytosphingosine** (0.0004 wt%) | **Absorbance** | **SD** |
|---|---|---|---|---|---|
| E | X | - | - | 0.81 | 0.05 |
| F | - | X | - | 0.87 | 0.13 |
| G | X | X | - | 0.82 | 0.04 |
| H | X | - | X | 0.79 | 0.02 |
| 2 | X | X | X | 0.11 | 0.004 |

**Table 3**

| **Ex.** | **Glycerol** (1 wt%) | **THPE** (2 wt%) | **Phytosphingosine** (0.0004 wt%) | **Absorbance** | **SD** |
|---|---|---|---|---|---|
| I | X | - | - | 0.67 | 0.07 |
| J | - | X | - | 0.56 | 0.23 |
| K | X | X | - | 0.76 | 0.04 |
| L | X | - | X | 0.45 | 0.12 |
| 3 | X | X | X | 0.11 | 0.002 |

**Table 4**

| **Ex.** | **Panthenol** (1 wt%) | **THPE** (2%) | **Phytosphingosine** (0.0004 wt%) | **Absorbance** | **SD** |
|---|---|---|---|---|---|
| M | X | - | - | 0.81 | 0.05 |
| N | - | X | - | 0.56 | 0.23 |
| O | X | X | - | 0.37 | 0.11 |
| P | X | - | X | 0.79 | 0.02 |
| 4 | X | X | X | 0.11 | 0.002 |

**Table 5**

| **Ex.** | **Glycerol** (1 wt%) + **Panthenol** (1 wt%) | **THPE** (1 wt%) | **Phytosphingosine** (0.0004 wt%) | **Absorbance** | **SD** |
|---|---|---|---|---|---|
| Q | X | - | - | 0.64 | 0.08 |
| R | - | X | - | 0.87 | 0.13 |
| S | X | X | - | 0.88 | 0.03 |
| T | X | - | X | 0.61 | 0.08 |
| 5 | X | X | X | 0.11 | 0.01 |

**Table 6**

| **Ex.** | **Glycerol** (1 wt%) + **Panthenol** (1 wt%) | **THPE** (2 wt%) | **Phytosphingosine** (0.0004 wt%) | **Absorbance** | **SD** |
|---|---|---|---|---|---|
| U | X | - | - | 0.64 | 0.08 |
| V | - | X | - | 0.56 | 0.23 |
| W | X | X | - | 0.57 | 0.13 |
| X | X | - | X | 0.61 | 0.08 |
| 6 | X | X | X | 0.11 | 0.002 |

The data in the tables 1 to 6 above, indicate that the composition as per the present invention provides for synergistic antimicrobial activity.

Further, the above experiments were repeated in exactly the same way except that sucrose (at 2 wt%) was used as the polyhydroxy compound instead of glycerol/panthenol. The data was also analysed in the same manner and the results are summarised in the table 7 and 8 below:

**Table 7**

| **Ex.** | **Sucrose** (2 wt%) | **THPE** (1 wt%) | **Phytosphingosine** (0.0004 wt%) | **Absorbance** | **SD** |
|---|---|---|---|---|---|
| Y | X | - | - | 0.84 | 0.07 |
| Z | X | X | - | 1.25 | 0.07 |
| AA | X | - | X | 0.46 | 0.08 |
| 7 | X | X | X | 0.13 | 0.001 |

**Table 8**

| **Ex.** | **Sucrose** (2 wt%) | **THPE** (2 wt%) | **Phytosphingosine** (0.0004 wt%) | **Absorbance** | **SD** |
|---|---|---|---|---|---|
| AB | X | - | - | 0.84 | 0.07 |
| AC | X | X | - | 1.47 | 0.07 |
| AD | X | - | X | 0.46 | 0.08 |
| 8 | X | X | X | 0.13 | 0.004 |

The data in Tables 7 and 8 above, indicates that the synergy is reproducible when the polyhydroxy compound is sucrose.

Further, the above experiments were repeated in exactly the same way except that fructose (2 wt%) was used as the polyhydroxy compound. The data was also analysed in the same manner and the results are summarised in the tables 9 and 10 below:

**Table 9**

| **Ex.** | **Fructose** (2 wt%) | **THPE** (1 wt%) | **Phytosphingosine** (0.0004 wt%) | **Absorbance** |
|---|---|---|---|---|
| AE | X | - | - | 0.68 |
| AF | X | X | - | 0.92 |
| AG | X | - | X | 0.29 |
| 9 | X | X | X | 0.04 |

**Table 10**

| **Ex.** | **Fructose** (2 wt%) | **THPE** (2 wt%) | **Phytosphingosine** (0.0004 wt%) | **Absorbance** |
|---|---|---|---|---|
| AH | X | - | - | 0.68 |
| Al | X | X | - | 0.98 |
| AJ | X | - | X | 0.29 |
| 10 | X | X | X | 0.05 |

The data in Tables 9 and 10 above, indicates that the synergy is reproducible when the polyhydroxy compound is fructose.

Further, the above experiments were repeated in exactly the same way except that fructose oligosaccharide (2 wt%) was used as the polyhydroxy compound. The data was also analysed in the same manner and the results are summarised in the tables 11 and 12 below:

**Table 11**

| **Ex.** | **FOS** (2 wt%) | **THPE** (1 wt%) | **Phytosphingosine** (0.0004 wt%) | **Absorbance** |
|---|---|---|---|---|
| AK | X | - | - | 0.55 |
| AL | X | X | - | 0.92 |
| AM | X | - | X | 0.29 |
| 11 | X | X | X | 0.05 |

**Table 12**

| **Ex.** | **FOS** (2 wt%) | **THPE** (2 wt%) | **Phytosphingosine** (0.0004 wt%) | **Absorbance** |
|---|---|---|---|---|
| AN | X | - | - | 0.55 |
| AO | X | X | - | 0.96 |
| AP | X | - | X | 0.29 |
| 12 | X | X | X | 0.05 |

The data in Tables 11 and 12 above, indicates that the synergy is reproducible when the polyhydroxy compound is fructose oligosaccharide.

Further, the above experiments were repeated in exactly the same way except that lauric acid (0.00025 wt%) was used instead of phytosphingosine as the antimicrobial lipid in combination with glycerol (table 13) or fructose (table 14) or sucrose (table 15) or fructose oliogosaccharide (table 16); and THPE. The data was also analysed in the same manner and the results are summarised in the table 13 to 16 below:

**Table 13**

| **Ex.** | **Glycerol** (2 wt%) | **THPE** (2 wt%) | **Lauric acid** (0.00025 wt%) | **Absorbance** |
|---|---|---|---|---|
| AQ | X | - | - | 0.65 |
| AR | X | X | - | 0.92 |
| AS | X | - | X | 0.63 |
| 13 | X | X | X | 0.37 |

**Table 14**

| **Ex.** | **Fructose** (2 wt%) | **THPE** (2 wt%) | **Lauric acid** (0.00025 wt%) | **Absorbance** |
|---|---|---|---|---|
| AT | X | - | - | 0.71 |
| AU | X | X | - | 0.94 |
| AV | X | - | X | 0.68 |
| 14 | X | X | X | 0.43 |

**Table 15**

| **Ex.** | **Sucrose** (2 wt%) | **THPE** (2 wt%) | **Lauric acid** (0.00025 wt%) | **Absorbance** |
|---|---|---|---|---|
| AW | X | - | - | 0.46 |
| AX | X | X | - | 0.75 |
| AY | X | - | X | 0.49 |
| 15 | X | X | X | 0.34 |

**Table 16**

| **Ex.** | **FOS** (2 wt%) | **THPE** (2 wt%) | **Lauric acid** (0.00025 wt%) | **Absorbance** |
|---|---|---|---|---|
| AZ | X | - | - | 0.48 |
| BA | X | X | - | 0.76 |
| BB | X | - | X | 0.51 |
| 16 | X | X | X | 0.30 |

The data in Tables 13 to 16 above, indicates that the synergy is reproducible when the the antimicrobial lipid is lauric acid. The data in these tables also show that synergy is obtained when antimicrobial lipid and THPE are present in the ratio range (1:10,000 to 1:10) as per the present invention; here 1:8000.

Further, the above experiments were repeated for glycerol as the polyhydroxy compound, phytosphingosine as the antimicrobial lipid; and THPE, where phytosphingosine and THPE were used in ratios as indicated in tables 17 to 22 below.

**Table 17**

| **Ex.** | **Glycerol** (2 wt%) | **THPE** (0.0004 wt%) | **Phytosphingosine** (0.0004 wt%) | **Absorbance** |
|---|---|---|---|---|
| BC | X | - | - | 0.65 |
| BD | X | X | - | 0.64 |
| BE | X | - | X | 0.34 |
| BF | X | X | X | 0.64 |

| | | | | |
|---|---|---|---|---|
| Phytosphingosine:THPE = 1:1 | | | | |

**Table 18**

| **Ex.** | **Glycerol** (2 wt%) | **THPE** (0.002 wt%) | **Phytosphingosine** (0.0004 wt%) | **Absorbance** |
|---|---|---|---|---|
| BG | X | - | - | 0.65 |
| BH | X | X | - | 0.63 |
| BI | X | - | X | 0.34 |
| BJ | X | X | X | 0.64 |

| | | | | |
|---|---|---|---|---|
| Phytosphingosine:THPE = 1:5 | | | | |

**Table 19**

| **Ex.** | **Glycerol** (2 wt%) | **THPE** (1 wt%) | **Phytosphingosine** (0.0004 wt%) | **Absorbance** |
|---|---|---|---|---|
| BK | X | - | - | 0.65 |
| BL | X | X | - | 0.95 |
| BM | X | - | X | 0.34 |
| 17 | X | X | X | 0.10 |

| | | | | |
|---|---|---|---|---|
| Phytosphingosine:THPE = 1:2500 | | | | |

**Table 20**

| **Ex.** | **Glycerol** (2 wt%) | **THPE** (2 wt%) | **Phytosphingosine** (0.0004 wt%) | **Absorbance** |
|---|---|---|---|---|
| BN | X | - | - | 0.65 |
| BO | X | X | - | 0.88 |
| BP | X | - | X | 0.34 |
| 18 | X | X | X | 0.10 |

| | | | | |
|---|---|---|---|---|
| Phytosphingosine:THPE = 1:5000 | | | | |

**Table 21**

| **Ex.** | **Glycerol** (2 wt%) | **THPE** (1 wt%) | **Phytosphingosine** (0.00004 wt%) | **Absorbance** |
|---|---|---|---|---|
| BQ | X | - | - | 0.65 |
| BR | X | X | - | 1.05 |
| BS | X | - | X | 0.63 |
| BT | X | X | X | 1.08 |

| | | | | |
|---|---|---|---|---|
| Phytosphingosine:THPE = 1:25,000 | | | | |

**Table 22**

| **Ex.** | **Glycerol** (2 wt%) | **THPE** (1 wt%) | **Phytosphingosine** (0.00002 wt%) | **Absorbance** |
|---|---|---|---|---|
| BU | X | - | - | 0.65 |
| BV | X | X | - | 1.05 |
| BW | X | - | X | 0.62 |
| BX | X | X | X | 1.10 |

| | | | | |
|---|---|---|---|---|
| Phytosphingosine:THPE = 1:50,000 | | | | |

The data in tables 17 to 22 above, show that synergy is obtained only when antimicrobial lipid and THPE were present in ratio as per the present invention.

## Claims

1. A topical composition comprising:
a) polyhydroxy compound selected from a saccharide, a polyol or combinations thereof;
b) antimicrobial lipid; and
c) tetrahydroxypropyl ethylenediamine (THPE),
wherein the antimicrobial lipid and THPE are present in a weight ratio from 1:10,000 to 1:10.

2. The topical composition according to claim 1, wherein the antimicrobial lipid is selected from lauric acid, sapienic acid, sphingosine, dihydrosphingosine, phytosphingosine, 6-hydroxy sphingosine, palmitoleic acid and combinations thereof.

3. The topical composition according to claim 2, wherein the antimicrobial lipid is phytosphingosine.

4. The topical composition according to any one of the preceding claims, wherein the composition contains 0.01 to 25 wt% of the polyhydroxy compound.

5. The topical composition according to any one of the preceding claims, wherein the composition contains 0.0001 to 5 wt% of the antimicrobial lipid.

6. The topical composition according to any one of the preceding claims, wherein the composition contains 0.01 to 10 wt% of THPE.

7. The topical composition according to any one of the preceding claims, wherein the polyhydroxy compound and THPE are present in a weight ratio that lies in the range of 1:20 to 20:1.

8. The topical composition according to any one of the preceding claims, wherein the composition is a lotion, a cream, a deodorant, a hand sanitizer or a body spray.

9. A non-therapeutic method of disinfecting a surface comprising the step of applying onto the surface a topical composition according to any one of claims 1 to 8.

10. The non-therapeutic method according to claim 9, wherein the surface is skin or hair.

11. The non-therapeutic method according to claim 9, wherein the surface is selected from the inner lining of the oral cavity, tongue and teeth.

12. The method according to any one of claims 9 to 11, wherein at least a part of the topical composition is removed after application onto the surface by rinsing said surface with water.

13. Non-therapeutic use of the topical composition according to any one of claims 1 to 8 for sanitising skin, hair or the oral cavity.

14. A topical composition according to any one of claims 1 to 8 for use in a therapeutic method of providing antimicrobial effect to a surface, wherein the surface is selected from skin, hair, oral cavity, tongue and teeth.

15. Use of a polyhydroxy compound selected from a saccharide, a polyol or combinations thereof, as an antimicrobial activity promoter in a composition comprising a combination of antimicrobial lipid and tetrahydroxypropyl ethylenediamine (THPE); the polyhydroxy compound preferably being selected from glycerol, panthenol, sucrose and combinations thereof.

## Patentansprüche

1. Topische Zusammensetzung, umfassend:
a) eine Polyhydroxyverbindung, ausgewählt unter einem Saccharid, einem Polyol oder Kombinationen davon;
b) ein antimikrobielles Lipid; und
c) Tetrahydroxypropylethylendiamin (THPE),
wobei das antimikrobielle Lipid und das THPE in einem Gewichtsverhältnis von 1:10.000 bis 1:10 vorliegen.

2. Topische Zusammensetzung nach Anspruch 1, wobei das antimikrobielle Lipid unter Laurinsäure, Sapiensäure, Sphingosin, Dihydrosphingosin, Phytosphingosin, 6-Hydroxysphingosin, Palmitoleinsäure und Kombinationen davon ausgewählt ist.

3. Topische Zusammensetzung nach Anspruch 2, wobei das antimikrobielle Lipid Phytosphingosin ist.

4. Topische Zusammensetzung nach irgendeinem der vorhergehenden Ansprüche, wobei die Zusammensetzung 0,01 bis 25 Gew.-% der Polyhydroxyverbindung enthält.

5. Topische Zusammensetzung nach irgendeinem der vorhergehenden Ansprüche, wobei die Zusammensetzung 0,0001 bis 5 Gew.-% des antimikrobiellen Lipids enthält.

6. Topische Zusammensetzung nach irgendeinem der vorhergehenden Ansprüche, wobei die Zusammensetzung 0,01 bis 10 Gew.-% THPE enthält.

7. Topische Zusammensetzung nach irgendeinem der vorhergehenden Ansprüche, wobei die Polyhydroxyverbindung und das THPE in einem Gewichtsverhältnis im Bereich von 1:20 bis 20:1 vorliegen.

8. Topische Zusammensetzung nach irgendeinem der vorhergehenden Ansprüche, wobei die Zusammensetzung eine Lotion, eine Creme, ein Deodorant, ein Handdesinfektionsmittel oder ein Körperspray ist.

9. Nicht-therapeutisches Verfahren zur Desinfektion einer Oberfläche, umfassend den Schritt des Auftragens einer topischen Zusammensetzung nach irgendeinem der Ansprüche 1 bis 8 auf die Oberfläche.

10. Nicht-therapeutisches Verfahren nach Anspruch 9, wobei die Oberfläche Haut oder Haar ist.

11. Nicht-therapeutisches Verfahren nach Anspruch 9, wobei die Oberfläche unter der Mundschleimhaut, der Zunge und den Zähnen ausgewählt ist.

12. Verfahren nach irgendeinem der Ansprüche 9 bis 11, wobei mindestens ein Teil der topischen Zusammensetzung nach dem Auftragen auf die Oberfläche durch Abspülen der Oberfläche mit Wasser entfernt wird.

13. Nicht-therapeutische Verwendung der topischen Zusammensetzung nach irgendeinem der Ansprüche 1 bis 8 zur Desinfektion von Haut, Haar oder der Mundhöhle.

14. Topische Zusammensetzung nach irgendeinem der Ansprüche 1 bis 8 zur Verwendung in einem therapeutischen Verfahren zur Erzielung einer antimikrobiellen Wirkung auf einer Oberfläche, wobei die Oberfläche unter Haut, Haar, Mundhöhle, Zunge und Zähnen ausgewählt ist.

15. Verwendung einer Polyhydroxyverbindung, ausgewählt unter einem Saccharid, einem Polyol oder Kombinationen davon, als Promotor der antimikrobiellen Aktivität in einer Zusammensetzung, die eine Kombination aus einem antimikrobiellen Lipid und Tetrahydroxypropylethylendiamin (THPE) umfasst, wobei die Polyhydroxyverbindung vorzugsweise aus Glycerin, Panthenol, Saccharose und Kombinationen davon ausgewählt ist.

## Revendications

1. Composition topique comprenant:
a) un composé polyhydroxylé choisi parmi un saccharide, un polyol ou une combinaison de ceux-ci;
b) un lipide antimicrobien; et
c) de la tétrahydroxypropyléthylènediamine (THPE),
où le lipide antimicrobien et la THPE sont présents dans un rapport pondéral de 1:10000 à 1:10.

2. Composition topique selon la revendication 1, où le lipide antimicrobien est choisi parmi l'acide laurique, l'acide sapiénique, la sphingosine, la dihydrosphingosine, la phytosphingosine, la 6-hydroxysphingosine, l'acide palmitoléique ou une combinaison de ceux-ci.

3. Composition topique selon la revendication 2, où le lipide antimicrobien est la phytosphingosine.

4. Composition topique selon l'une quelconque des revendications précédentes, où la composition contient de 0,01 à 25 % en poids de composé polyhydroxylé.

5. Composition topique selon l'une quelconque des revendications précédentes, où la composition contient de 0,0001 à 5 % en poids de lipide antimicrobien.

6. Composition topique selon l'une quelconque des revendications précédentes, où la composition contient de 0,01 à 10 % en poids de THPE.

7. Composition topique selon l'une quelconque des revendications précédentes, où le composé polyhydroxylé et la THPE sont présents dans un rapport pondéral qui est situé dans la plage de 1:20 à 20:1.

8. Composition topique selon l'une quelconque des revendications précédentes, où la composition est une lotion, une crème, un déodorant, un désinfectant pour les mains ou un spray corporel.

9. Procédé non thérapeutique de désinfection d'une surface comprenant l'étape d'application sur la surface d'une composition topique selon l'une quelconque des revendications 1 à 8.

10. Procédé non thérapeutique selon la revendication 9, où la surface est la peau ou les cheveux.

11. Procédé non thérapeutique selon la revendication 9, où la surface est choisie parmi le revêtement interne de la cavité buccale, la langue et les dents.

12. Procédé selon l'une quelconque des revendications 9 à 11, où au moins une partie de la composition topique est éliminée après application sur la surface par rinçage de ladite surface à l'eau.

13. Utilisation non thérapeutique de la composition topique selon l'une quelconque des revendications 1 à 8 pour la désinfection de la peau, des cheveux ou de la cavité buccale.

14. Composition topique selon l'une quelconque des revendications 1 à 8 destinée à être utilisée dans un procédé thérapeutique pour conférer un effet antimicrobien à une surface, où la surface est choisie parmi la peau, les cheveux, la cavité buccale, la langue et les dents.

15. Utilisation d'un composé polyhydroxylé choisi parmi un saccharide, un polyol ou des combinaisons de ceux-ci comme promoteur d'activité antimicrobienne dans une composition comprenant une combinaison de lipide antimicrobien et de tétrahydroxypropyléthylènediamine (THPE); le composé polyhydroxylé étant de préférence choisi parmi le glycérol, le panthénol, le saccharose et des combinaisons de ceux-ci.
